# EUROPEAN PATENT APPLICATION

(11) **EP 1 195 431 A1**
(43) Date of publication of application: **10.04.2002**
(21) Application number: 00935595.9
(22) Date of filing: 08.06.2000
(51) Int. Cl.: C12N 1/21, C12P 13/08, C12N 15/54

(54) **PROCESS FOR PRODUCING L-LYSINE**

(30) Priority: 15.06.1999 JP 16837799; 01.11.1999 JP 31111199
(71) Applicant: Ajinomoto Co., Inc., Tokyo 104-8315 (JP)
(72) Inventor: SUGIMOTO, Masakazu, Ajinomoto Co., Inc., Kawasaki-shi, Kanagawa 210-868 (JP); TAKAMURA, Jun, Ajinomoto Co., Inc., Kawasaki-shi, Kanagawa 210-868 (JP); IZUI, Hiroshi, Ajinomoto Co., Inc., Kawasaki-shi, Kanagawa 210-868 (JP); KIMURA, Eiichiro, Ajinomoto Co., Inc., Kawasaki-shi, Kanagawa 210-868 (JP); ITO, Hisao, Ajinomoto Co., Inc., Kawasaki-shi, Kanagawa 210-868 (JP); NAKAMATSU, Tsuyoshi, Ajinomoto Co., Inc., Kawasaki-shi, Kanagawa 210-868 (JP); KURAHASHI, Osamu, Ajinomoto Co., Inc., Kawasaki-shi, Kanagawa 210-868 (JP)
(74) Representative: Strehl Schübel-Hopf & Partner
(86) International application number: JP0003736
(87) International publication number: WO0077172

(57) **Abstract**

A coryneform bacterium in which an intracellular activity of 6-phosphofructokinase is increased, and which has L-lysine productivity, and a method for producing L-lysine comprising the steps of cultivating the coryneform bacterium, to produce and accumulate L-lysine in a culture, and collecting L-lysine from the culture, as well as a DNA usable in increasing the 6-phosphofructokinase activity.

Sequence Listing

<110> Ajinomoto Co., Inc.

<120> Method for producing L-lysine

<130> EPA-53789

<150> JP 11-311111
<151> 1999-11-01

<150> JP 11-168377
<151> 1999-06-15

<160> 12

<170> PatentIn Ver. 2.0

<210> 1
<211> 24
<212> DNA
<213> Artificial Sequence

<220>
<223> Description of Artificial Sequence: primer for amplifying Escherichia
coli pfkB gene

<400> 1
aagcttcatt tatcaagagt ccgt 24

<210> 2
<211> 24
<212> DNA
<213> Artificial Sequence

<220>
<223> Description of Artificial Sequence: primer for amplifying Escherichia
coli pfkB gene

<400> 2
tgattccccc aatgctgggg gttt                                                24

<210> 3
<211> 17
<212> DNA
<213> Artificial Sequence

<220>
<223> Description of Artificial Sequence: primer 1 for pfk cloning

<220>
<221> misc_feature
<222> (3)
<223> n=a or g or c or t

<400> 3
acnathgaya aygayrt                                                        17

<210> 4
<211> 20
<212> DNA
<213> Artificial Sequence

<220>
<223> Description of Artificial Sequence: primer 2 for pfk cloning

<220>
<221> misc_feature
<222> (3,6,9,15)
<223> n=a or g or c or t

<400> 4
gtnccnccnc kytgnayrtg                                                     20

<210> 5
<211> 16

<212> DNA
<213> Artificial Sequence

<220>
<223> Description of Artificial Sequence: sequence primer M13-20

<400> 5
gtaaaacgac ggccag                                                    16

<210> 6
<211> 17
<212> DNA
<213> Artificial Sequence

<220>
<223> Description of Artificial Sequence: sequence primer M13RV

<400> 6
caggaaacag ctatgac                                                   17

<210> 7
<211> 20
<212> DNA
<213> Artificial Sequence

<220>
<223> Description of Artificial Sequence: Inverse PCR primer 1A

<400> 7
agcaatccaa cccacgtggc                                                20

<210> 8
<211> 20
<212> DNA
<213> Artificial Sequence

<220>
<223> Description of Artificial Sequence: Inverse PCR primer 1B

<400> 8
acattgacca gttcggtcac                                                    20

<210> 9
<211> 20
<212> DNA
<213> Artificial Sequence

<220>
<223> Description of Artificial Sequence: nested primer A

<400> 9
ggcccatgac ctccacgatc                                                    20

<210> 10
<211> 20
<212> DNA
<213> Artificial Sequence

<220>
<223> Description of Artificial Sequence: nested primer B

<400> 10
gaccttcacg ggaattggac                                                    20

<210> 11
<211> 1357
<212> DNA
<213> Brevibacterium lactofermentum

<220>
<221> CDS
<222> (116)..(1153)

<400> 11
ctgcagctct ggcgattaaa taaggtggtc agagacaatt tttcggcctg tcaacccctg 60
tgattctctt atttttgggt gattgttccg gcgcgggtgt tgtgatgggt ttaat atg  118

Met
1
gaa gac atg cga att gct act ctc acg tca ggc ggc gac tgc ccc gga 166
Glu Asp Met Arg Ile Ala Thr Leu Thr Ser Gly Gly Asp Cys Pro Gly
            5                   10                  15
cta aat gcc gtc atc cga gga atc gtc cgc aca gcc agc aat gaa ttt 214
Leu Asn Ala Val Ile Arg Gly Ile Val Arg Thr Ala Ser Asn Glu Phe
        20                  25                  30
ggc tcc acc gtc gtt ggt tat caa gac ggt tgg gaa gga ctg tta gcc 262
Gly Ser Thr Val Val Gly Tyr Gln Asp Gly Trp Glu Gly Leu Leu Ala
    35                  40                  45
gat cgt cgc gta cag ctg tat gac gat gaa gat att gac cga atc ctc 310
Asp Arg Arg Val Gln Leu Tyr Asp Asp Glu Asp Ile Asp Arg Ile Leu
50                  55                  60                  65
ctt cga ggc ggc acc att ttg ggc act ggt cgc ctc cat ccg gac aag 358
Leu Arg Gly Gly Thr Ile Leu Gly Thr Gly Arg Leu His Pro Asp Lys
                70                  75                  80
ttt aag gcc gga att gat cag att aag gcc aac tta gaa gac gcc ggc 406
Phe Lys Ala Gly Ile Asp Gln Ile Lys Ala Asn Leu Glu Asp Ala Gly
            85                  90                  95
atc gat gcc ctt atc cca atc ggt ggc gaa gga acc ctg aag ggt gcc 454
Ile Asp Ala Leu Ile Pro Ile Gly Gly Glu Gly Thr Leu Lys Gly Ala
        100                 105                 110
aag tgg ctg tct gat aac ggt atc cct gtt gtc ggt gtc cca aag acc 502
Lys Trp Leu Ser Asp Asn Gly Ile Pro Val Val Gly Val Pro Lys Thr
    115                 120                 125
att gac aat gac gtg aat ggc act gac ttc acc ttc ggt ttc gat act 550
Ile Asp Asn Asp Val Asn Gly Thr Asp Phe Thr Phe Gly Phe Asp Thr
130                 135                 140                 145
gct gtg gca gtg gct acc gac gct gtt gac cgc ctg cac acc acc gct 598
Ala Val Ala Val Ala Thr Asp Ala Val Asp Arg Leu His Thr Thr Ala
                150                 155                 160
gaa tct cac aac cgt gtg atg atc gtg gag gtc atg ggc cgc cac gtg 646
Glu Ser His Asn Arg Val Met Ile Val Glu Val Met Gly Arg His Val
            165                 170                 175
ggt tgg att gct ctg cac gca ggt atg gcg ggc ggt gct cac tac acc 694
Gly Trp Ile Ala Leu His Ala Gly Met Ala Gly Gly Ala His Tyr Thr
        180                 185                 190

gtt att cca gaa gta cct ttc gat att gca gag atc tgc aag gcg atg 742
Val Ile Pro Glu Val Pro Phe Asp Ile Ala Glu Ile Cys Lys Ala Met
    195                 200                 205
gaa cgt cgc ttc cag atg ggc gag aag tac ggc att atc gtc gtt gcg 790
Glu Arg Arg Phe Gln Met Gly Glu Lys Tyr Gly Ile Ile Val Val Ala
210                 215                 220                 225
gaa ggt gcg ttg cca cgc gaa ggc acc atg gag ctt cgt gaa ggc cac 838
Glu Gly Ala Leu Pro Arg Glu Gly Thr Met Glu Leu Arg Glu Gly His
                230                 235                 240
att gac cag ttc ggt cac aag acc ttc acg gga att gga cag cag atc 886
Ile Asp Gln Phe Gly His Lys Thr Phe Thr Gly Ile Gly Gln Gln Ile
            245                 250                 255
gct gat gag atc cac gtg cgc ctc ggc cac gat gtt cgt acg acc gtt 934
Ala Asp Glu Ile His Val Arg Leu Gly His Asp Val Arg Thr Thr Val
        260                 265                 270
ctt ggc cac att caa cgt ggt gga acc cca act gct ttc gac cgt gtt 982
Leu Gly His Ile Gln Arg Gly Gly Thr Pro Thr Ala Phe Asp Arg Val
    275                 280                 285
ctg gcc act cgt tat ggt gtt cgt gca gct cgt gcg tgc cat gag gga 1030
Leu Ala Thr Arg Tyr Gly Val Arg Ala Ala Arg Ala Cys His Glu Gly
290                 295                 300                 305
agc ttt gac aag gtt gtt gct ttg aag ggt gag agc att gag atg atc 1078
Ser Phe Asp Lys Val Val Ala Leu Lys Gly Glu Ser Ile Glu Met Ile
                310                 315                 320
acc ttt gaa gaa gcc gtc gga acc ttg aag gaa gtc cca ttc gaa cgc 1126
Thr Phe Glu Glu Ala Val Gly Thr Leu Lys Glu Val Pro Phe Glu Arg
            325                 330                 335
tgg gtt act gcc cag gca atg ttt gga tagtttttcg ggcttttatc 1173
Trp Val Thr Ala Gln Ala Met Phe Gly
        340                 345
aacagccaat aacagctctt tcgctcattg aggtgaaggg gctgtttttt catgccgtaa 1233
ggaaagtgca agtaagtgaa atcaagtggc atagatccat tgatgcttag actgtgacct 1293
aggcttgact ttcgtggggg agtggggata agttcatctt aaacacaatg caatcgattg 1353
catt 1357

<210> 12
<211> 346
<212> PRT

<213> Brevibacterium lactofermentum

<400> 12
Met Glu Asp Met Arg Ile Ala Thr Leu Thr Ser Gly Gly Asp Cys Pro
1               5                   10                  15
Gly Leu Asn Ala Val Ile Arg Gly Ile Val Arg Thr Ala Ser Asn Glu
            20                  25                  30
Phe Gly Ser Thr Val Val Gly Tyr Gln Asp Gly Trp Glu Gly Leu Leu
        35                  40                  45
Ala Asp Arg Arg Val Gln Leu Tyr Asp Asp Glu Asp Ile Asp Arg Ile
    50                  55                  60
Leu Leu Arg Gly Gly Thr Ile Leu Gly Thr Gly Arg Leu His Pro Asp
65                  70                  75                  80
Lys Phe Lys Ala Gly Ile Asp Gln Ile Lys Ala Asn Leu Glu Asp Ala
                85                  90                  95
Gly Ile Asp Ala Leu Ile Pro Ile Gly Gly Glu Gly Thr Leu Lys Gly
            100                 105                 110
Ala Lys Trp Leu Ser Asp Asn Gly Ile Pro Val Val Gly Val Pro Lys
        115                 120                 125
Thr Ile Asp Asn Asp Val Asn Gly Thr Asp Phe Thr Phe Gly Phe Asp
    130                 135                 140
Thr Ala Val Ala Val Ala Thr Asp Ala Val Asp Arg Leu His Thr Thr
145                 150                 155                 160
Ala Glu Ser His Asn Arg Val Met Ile Val Glu Val Met Gly Arg His
                165                 170                 175
Val Gly Trp Ile Ala Leu His Ala Gly Met Ala Gly Gly Ala His Tyr
            180                 185                 190
Thr Val Ile Pro Glu Val Pro Phe Asp Ile Ala Glu Ile Cys Lys Ala
        195                 200                 205
Met Glu Arg Arg Phe Gln Met Gly Glu Lys Tyr Gly Ile Ile Val Val
    210                 215                 220
Ala Glu Gly Ala Leu Pro Arg Glu Gly Thr Met Glu Leu Arg Glu Gly
225                 230                 235                 240
His Ile Asp Gln Phe Gly His Lys Thr Phe Thr Gly Ile Gly Gln Gln
                245                 250                 255
Ile Ala Asp Glu Ile His Val Arg Leu Gly His Asp Val Arg Thr Thr
            260                 265                 270
Val Leu Gly His Ile Gln Arg Gly Gly Thr Pro Thr Ala Phe Asp Arg

        275                 280                 285
Val Leu Ala Thr Arg Tyr Gly Val Arg Ala Ala Arg Ala Cys His Glu
    290                 295                 300
Gly Ser Phe Asp Lys Val Val Ala Leu Lys Gly Glu Ser Ile Glu Met
305                 310                 315                 320
Ile Thr Phe Glu Glu Ala Val Gly Thr Leu Lys Glu Val Pro Phe Glu
                325                 330                 335
Arg Trp Val Thr Ala Gln Ala Met Phe Gly
            340                 345

## Description

### Technical Field

The present invention relates to a method for producing L-lysine by fermentation. L-Lysine is widely used as a fodder additive or the like.

### Background Art

L-Lysine has been industrially produced by fermentation by using coryneform bacteria belonging to the genus *Brevibacterium* or *Corynebacterium*, which have L-lysine productivity. As these coryneform bacteria, strains isolated from the nature world or artificial mutants of the strains have been used.

There have been disclosed various technologies to increase the L-lysine productivity by increasing activities of L-lysine biosynthesis enzymes by recombinant DNA technology. For example, it is known that in coryneform bacteria having L-lysine productivity, the L-lysine productivity of the bacteria is improved by introduction of a gene encoding aspartokinase desensitized in feedback inhibition by L-lysine and L-threonine (mutant *lysC*), a dihydrodipicolinate reductase gene (*dapB*), a dihydrodipicolinate synthase gene (*dapA*), a diaminopimelate decarboxylase gene (*lysA*), and a diaminopimelate dehydrogenase gene (*ddh*) (WO 96/40934); *lysA* and *ddh* (Japanese Patent Application Laid-Open No. 9-322774); *lysC, lysA* and a phosphoenolpyruvate carboxylase gene (*ppc*) (Japanese Patent Application Laid-Open No. 10-165180); or mutant *lysC, dapB, dapA*, *lysA* and an aspartate aminotransferase gene (*aspC*) (Japanese Patent Application Laid-Open No. 10-215883).

On the other hand, it is known that L-glutamic acid productivity of coryneform bacteria is improved when 6-phosphofructokinase (hereinafter, also simply referred to as "phosphofructokinase") originating from *Escherichia coli* is introduced into the bacteria (Japanese Patent Application Laid-Open No. 63-102692).

However, no relationship between phosphofructokinase activity and L-lysine productivity has been known.

In addition, a structure of a gene encoding phosphofructokinase from coryneform bacteria has not been known.

### Disclosure of Invention

An object of the present invention is to provide an efficient method for producing L-lysine by fermentation, and a bacterium and a DNA usable in the method.

The present inventors earnestly studied to solve the above problems. As a result, they found that a production amount of L-lysine can be improved by introducing a gene encoding phosphofructokinase into coryneform bacteria to increase phosphofructokinase activity. Also, they succeeded in isolating a novel phosphofructokinase gene from *Brevibacterium lactofermentum*. Based on this finding, the present invention has been completed.

Thus the present invention provides the followings.
(1) A coryneform bacterium in which an intracellular activity of 6-phosphofructokinase is increased, and which has L-lysine productivity.
(2) The coryneform bacterium according to (1), wherein the intracellular activity of 6-phosphofructokinase is increased by increasing a number of copies of a gene encoding 6-phosphofructokinase in a cell of the bacterium.
(3) The coryneform bacterium according to (2), wherein the gene encoding 6-phosphofructokinase originates from a coryneform bacterium.
(4) A method for producing L-lysine comprising the steps of cultivating said coryneform bacterium as defined in any one of (1) to (3) in a medium, to produce and accumulate L-lysine in a culture, and collecting L-lysine from the culture.
(5) A DNA which encodes a protein defined in the following (A) or (B):
   (A) a protein which has the amino acid sequence of SEQ ID NO: 12, or
   (B) a protein which has an amino acid sequences of SEQ ID NO: 12 including substitution, deletion, insertion, addition or inversion of one or several amino acids, and has 6-phosphofructokinase activity.
(6) The DNA according to (5), which is a DNA defined in the following (a) or (b):
   (a) a DNA which has a nucleotide sequence comprising the nucleotide sequence of the nucleotide numbers 116 to 1153 of SEQ ID NO: 11; or
   (b) a DNA which is hybridizable with a probe having the nucleotide sequence of the nucleotide numbers 116 to 1153 of SEQ ID NO: 11 or a part thereof under a stringent condition, and encodes a protein having 6-phosphofructokinase activity.

### Best Mode for Carrying Out the Invention

The present invention will be described below in detail.

### <1> The coryneform bacterium of the present invention

The coryneform bacterium of the present invention is a coryneform bacterium which has L-lysine productivity, and in which intracellular phosphofructokinase activity is increased. The phosphofructokinase activity is a catalyzing activity of a reaction of transferring a phosphate group to the position 1 of fructose 6-phosphate.

The coryneform bacteria referred to in the present invention are a group of microorganisms as defined in Bergey's Manual of Determinative Bacteriology, 8th ed., p. 599 (1974), which are aerobic Gram-positive non-acid-fast rods having no spore-forming ability. The coryneform bacteria include bacteria belonging to the genus *Brevibacterium* having been hitherto classified into the genus *Brevibacterium* but united as bacteria belonging to the genus *Corynebacterium* at present (*Int. J. Syst. Bacteriol.*, 41, 255 (1981)), and bacteria belonging to the genus *Brevibacterium* and the genus *Microbacterium* closely relative to bacteria belonging to the genus *Corynebacterium*. Examples of the coryneform bacterium suitably used to produce L-lysine include, for example, the following strains:
*Corynebacterium acetoacidophilum* ATCC 13870;
*Corynebacterium acetoglutamicum* ATCC 15806;
*Corynebacterium callunae* ATCC 15991;
*Corynebacterium glutamicum* ATCC 13032;
(*Brevibacterium divaricatum*) ATCC 14020;
(*Brevibacterium lactofermentum*) ATCC 13869;
*(Corynebacterium lilium*) ATCC 15990;
(*Brevibacterium flavum*) ATCC 14067;
*Corynebacterium melassecola* ATCC 17965;
*Brevibacterium saccharolyticum* ATCC 14066;
*Brevibacterium immariophilum* ATCC 14068;
*Brevibacterium roseum* ATCC 13825;
*Brevibacterium thiogenitalis* ATCC 19240;
*Microbacterium ammoniaphilum* ATCC 15354;
*Corynebacterium thermoaminogenes* AJ12340 (FERM BP-1539).

These can be furnished from, for example, the American Type Culture Collection. Each microorganism is assigned its registration number,and one can request provision of each microorganism by referring to its registration number. The registration numbers corresponding to the microorganisms are described in a catalog of the American Type Culture Collection. The AJ12340 strain has been deposited in National Institute of Bioscience and Human Technology, Agency of Industrial Science and Technology, Ministry of International Trade and Industry based on the Budapest Treaty.

Other than the bacterial strains described above, mutant strains having L-lysine productivity derived from these strains may be used for the present invention. Such artificial mutant strains include the followings:
S-(2-aminoethyl)-cysteine (hereinafter abbreviated as "AEC") resistant mutant strains (For example, *Brevibacterium lactofermentum* AJ11082 (NRRL B-11470); see Japanese Patent Publication Nos. 56-1914, 56-1915, 57-14157, 57-14158, 57-30474, 58-10075, 59-4993, 61-35840, 62-24074, 62-36673, 5-11958, 7-112437, and 7-112438); mutant strains which require an amino acid such as L-homoserine for their growth (Japanese Patent Publication Nos. 48-28078 and 56-6499); mutant strains which exhibit resistance to AEC and require amino acids such as L-leucine, L-homoserine, L-proline, L-serine, L-arginine, L-alanine, and L-valine (United States Patent Nos. 3,708,395 and 3,825,472); L-lysine-producing mutant strains which exhibit resistance to DE-α-amino-ε-caprolactam, α-amino-lauryllactam, aspartate-analog, sulfa drug, quinoid, and N-lauroylleucine; L-lysine-producing mutant strains which exhibit resistance to inhibitors of oxyaloacetate decarboxylase or respiratory system enzymes (Japanese Patent Application Laid-open Nos. 50-53588, 50-31093, 52-102498, 53-9394, 53-86089, 55-9783, 55-9759, 56-32995 and 56-39778, and Japanese Patent Publication Nos. 53-43591 and 53-1833); L-lysine-producing mutant strains which require inositol or acetic acid (Japanese Patent Application Laid-open Nos. 55-9784 and 56-8692); L-lysine-producing mutant strains which exhibit sensitivity to fluoropyruvic acid or temperature not less than 34°C (Japanese Patent Application Laid-open Nos. 55-9783 and 53-86090); and mutant strains belonging to the genus *Brevibacterium* or *Corynebacterium* which exhibit resistance to ethylene glycol and produce L-lysine (United States Patent No. 4,411,997).

The terms "L-lysine productivity" used herein means an ability to accumulate a significant amount of L-lysine in a medium when the coryneform bacterium is cultured in the medium or to increase L-lysine content in cells.

### <2> Increase of phosphofructokinase activity

In order to increase the phosphofructokinase activity in a coryneform bacterial cell, a recombinant DNA can be prepared by ligating a fragment of a gene encoding phosphofructokinase with a vector functioning in the bacterium, preferably a multi-copy vector, and introduced into a coryneform bacterium having L-lysine productivity to transform the bacterium. The copy number of the gene encoding phosphofructokinase in the cell of the transformant is increased, and as a result, the phosphofructokinase activity is increased. In *E. coli*, the phosphofructokinase is encoded by a *pfkA* gene and a *pfkB* gene.

As the gene encoding phosphofructokinase, those originating from coryneform bacteria as well as those originating from other organism such as bacteria belonging to the genus *Escherichia* may be used.

The nucleotide sequence of a gene encoding phosphofructokinase (*pfkB* gene) originating from *E. coli* has already been elucidated (Daldal, F., Gene, 28, 337-342 (1984); Daldal, F., J. Mol. Biol., 168,, 285-305 (1983); GenBank/EMBL/DDBJ accession No. K00128). Therefore, the *pfkB* gene can be obtained by PCR (polymerase chain reaction; see White, T.J. et al., Trends Genet. 5, 185 (1989)) utilizing primers prepared based on the nucleotide sequence, for example, primers shown in SEQ ID NOS: 1 and 2 in Sequence Listing and *E. coli* chromosome DNA as a template. Because the *pfkA* gene originating from *E. coli* has also already been elucidated (Eur. J. Biochem., 149(2), 363-373 (1985)), the *pfkA* gene may be obtained similarly. Phosphofructokinase-encoding genes originating from other microorganisms such as coryneform bacteria may be obtained similarly.

The chromosome DNA can be prepared from a bacterium, which is a DNA donor, by the method of Saito and Miura (H. Saito and K. Kimura, Biochem. Biophys. Acta, 72, 619 (1963); Seibutsu Kogaku Jikkensho, The Society for Bioscience and Bioengineering, Japan ed., 97-98, Baifukan, 1992), for example.

As to a gene encoding phosphofructokinase (*pfk* gene) from a coryneform bacterium, a part thereof can be isolated by selecting a region having a high homology among amino acid sequences deduced from nucleotide sequences of known phosphofructokinase-encoding genes such as the *pfkA* gene from *Escherichia coli* (Eur. J. Biochem. 149(2), 363-373 (1985)) and a *pfk* gene from *Streptomyces coelicolor* (Appl. Environ. Microbiol. 63(3), 956-961 (1997)), and conducting PCR using primers prepared based on an amino acid sequence of the region. Examples of the primers include oligonucleotides shown in SEQ ID NO: 3 and SEQ ID NO: 4.

In order to determine the full-length sequence of the *pfk* gene by using the *pfk* gene fragment obtained as described above, the inverse PCR method (Genetics 120, 621-623 (1988)), a method using the LA-PCR in vitro cloning kit (Takara Shuzo) or the like is used. The inverse PCR method was adopted in the Examples described later. Specifically, PCR is conducted by using, for example, oligonucleotides shown in SEQ ID NO: 7 and SEQ ID NO: 8 as primers, and *Brevibacterium lactofermentum* chromosomal DNA as a template. Then nested PCR (Technique, 1, 165-170 (1989)) is conducted by using the obtained amplified product as a template, and oligonucleotides shown in SEQ ID NO: 9 and SEQ ID NO: 10. A nucleotide sequence of about 1300 bp containing the *pfk* gene in the nucleotide sequence determined as described above, is shown in SEQ ID NO: 11. A sequence obtained by translation of open reading frame predicted from the nucleotide sequence, to an amino acid sequence, is shown in SEQ ID NO: 12. Homology comparison with known sequences showed that this *pfk* gene from *Brevibacterium lactofermentum* is a novel gene. A DNA fragment containing the coding region of the gene can be also obtained by conducting PCR using nucleotide sequences of 5'- and 3'-untranslated regions in the nucleotide sequence shown in SEQ ID NO: 11, besides the method as described above.

The gene encoding phosphofructokinase may be a gene encoding phosphofructokinase having an amino acid sequence of SEQ ID NO: 12 including substitution, deletion, insertion, addition or inversion of one or several amino acids at one or several positions, unless the activity of the encoded phosphofructokinase is spoiled. The term "several" used herein may vary depending on position in the steric structure of the protein and kind of the amino acid residue. This is because some amino acids have their similar amino acids as isoleucine and valine, and the difference between the amino acids does not largely affect the steric structure of the protein. For example, phosphofructokinase encoded by the *pfk* gene from *Brevibacterium lactofermentum* may be one having the phosphofructokinase activity and having a homology of not less than 70 to 80%, preferably not less than 80 to 90% with respect to the entire amino acid sequence of SEQ ID NO: 11. The homology used herein is a value calculated by Lipman-Pearson method (Science 227, 1435-1441 (1985)) or Takashi & Gotoh method (J. Biochem. 92, 1173-1177 (1984)). Specifically, the term "several" may be 2 to 30, preferably 2 to 20, more preferably 2 to 10.

The DNA which encodes the substantially same protein as phosphofructokinase as mentioned above can be obtained by modifying a nucleotide sequence of a *pfk* gene so that the amino acid sequence should contain substitution, deletion, insertion, addition or inversion of an amino acid residue or residues at a particular site by, for example, site-specific mutagenesis. Such a modified DNA as mentioned above may also be obtained by a conventional mutagenesis treatment. Examples of the mutagenesis treatment include in vitro treatment of DNA encoding phosphofructokinase with hydroxylamine or the like, treatment of a microorganism such as an *Escherichia* bacterium, containing a gene encoding phosphofructokinase by UV irradiation or with mutagenesis agents used for usual mutagenesis treatment such as N-methyl-N'-nitro-N-nitrosoguanidine (NTG) and nitrous acid.

The aforementioned substitution, deletion, insertion, addition or inversion of nucleotides includes naturally occurring mutations (mutant or variant) such as those observed depending difference between species or strains of microorganisms containing phosphofructokinase.

The DNA which encodes substantially the same protein as phosphofructokinase described above can be obtained by allowing expression of a DNA having such a mutation as mentioned above in a suitable cell, and examining the phosphofructokinase activity of the expression product. The DNA which encodes substantially the same protein as phosphofructokinase can also be obtained by isolating, from a DNA encoding phosphofructokinase which have mutations or cells containing each of them, a DNA hybridizable under a stringent condition, for example, with a DNA comprising the nucleotide sequence of the nucleotide numbers 116-1153 of the nucleotide sequence shown by SEQ ID NO: 12 in the Sequence Listing or an oligonucleotide probe which can be prepared from the DNA by PCR or the like, and encoding a protein having phosphofructokinase activity. The term "stringent condition" used herein means a condition that allows formation of so-called specific hybrid and does not allow formation of non-specific hybrid. It is difficult to definitely show this condition by values. However, it may be, for example, a condition that allows hybridization of highly homologous DNA such as DNA having homology of 70% or higher, but does not allow hybridization of DNA of lower homology than defined above, or a washing condition of usual Southern hybridization, of a temperature of 60°C, 1 x SSC and 0.1% SDS, preferably 0.1 x SSC and 0.1% SDS. The homology used herein is a value calculated by Lipman-Pearson method (Science 227, 1435-1441 (1985)) or Takashi & Gotoh method (J. Biochem. 92, 1173-1177 (1984)). Design of the probe can be conducted according to the method known to one skilled in the art.

Genes that hybridize under such a condition as mentioned above also include those having a stop codon occurring in its sequence and those encoding an enzyme no longer having its activity due to a mutation of active center. However, such genes can readily be eliminated by ligating the genes to a commercially available activity expression vector, and measuring phosphofructokinase activity. The phosphofructokinase activity can be determined by the method described in, for example, Bloxham, D.P. and Lardy, H.A., The Enzymes (3^{rd} ed.), 8, 239-278 (1973).

It is preferred that the gene encoding phosphofructokinase obtained as described above is ligated with a vector DNA autonomously replicable in a cell of *E. coli* and/or coryneform bacteria to form a recombinant DNA, and the recombinant DNA is introduced into an *E. coli* cell, whereby the subsequent procedures can be made easy. The vector autonomously replicable in *E. coli* cells is preferably a plasmid vector, and preferably one autonomously replicable in a host cell. Examples thereof include pUC19, pUC18, pBR322, pHSG299, pHSG399, pHSG398, RSF1010 and so forth.

As a vector autonomously replicable in cells of coryneform bacteria, pAM330 (Japanese Patent Application Laid-Open No. 58-67699), pHM1519 (Japanese Patent Application Laid-Open No. 58-77895) and the like may be mentioned. Also, when a DNA fragment having ability to make a plasmid to be autonomously replicable in coryneform bacteria is taken out from these vectors and inserted into the above-mentioned vectors for *E. coli*, they can be used as a shuttle vector autonomously replicable in both of *E. coli* and coryneform bacteria. Examples of the shuttle vector include the followings. The accession numbers of international depositary authorities, for microorganisms harboring the respective vectors are shown in parentheses.
pAJ655: *Escherichia coli* AJ11882 (FERM BP-136) *corynebacterium glutamicum* SR8201 (ATCC 39135)
pAJ1844: *Escherichia coli* AJ11883 (FERM BP-137) *Corynebacterium glutamicum* SR8202 (ATCC 39136)
pAJ611: *Escherichia coli* AJ11884 (FERM BP-138)
pAJ3148: *Corynebacterium glutamicum* SR8203 (ATCC 39137)
pAJ440: *Bacillus subtilis* AJ11901 (FERM BP-140)
pHC4: *Escherichia coli* AJ12617 (FERM BP-3532)

In order to prepare recombinant DNA by ligating the phosphofructokinase gene with a vector, the vector is digested by a restriction enzyme corresponding to the termini of a DNA fragment containing the phosphofructokinase gene. Ligation is usually performed by using a ligase such as T4 DNA ligase.

To introduce the recombinant DNA prepared as described above into a coryneform bacterium, any known transformation methods can be employed. For example, a method of treating recipient cells with calcium chloride so as to increase the permeability of DNA, which has been reported for *Escherichia coli* K-12 (Mandel, M. and Higa, A., J. Mol. Biol., 53, 159 (1970)) and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)) may be used. Alternatively, a method of making DNA-recipient cells to be protoplasts or spheroplasts which can easily take up recombinant DNA followed by introducing the recombinant DNA thereinto (Chang, S. and Choen, S.N., Molec. Gen. Genet., 168, 111 (1979); Bibb,M.J., Ward, J.M. and Hopwood, O.A., Nature, 274, 398 (1978); Hinnen, A., Hicks, J.B. and Fink, G.R., Proc. Natl. Acad. Sci. USA, 75 1929 (1978)) may be also used. Also, the transformation of the coryneform bacteria may be performed according to the electric pulse method (Japanese Patent Application Laid-Open No. 2-207791).

Increase of the phosphofructokinase activity can also be achieved by allowing multiple copies of the phosphofructokinase-encoding gene to be present on the chromosomal DNA of the host. In order to introduce multiple copies of the phosphofructokinase-encoding gene into the chromosomal DNA of microorganisms belonging to the coryneform bacteria, the homologous recombination is carried out using a sequence whose multiple copies exist in the chromosomal DNA as targets. As the sequence whose multiple copies exist in the chromosomal DNA, repetitive DNA, inverted repeats existing at the end of a transposable element can be used. Alternatively, as disclosed in Japanese Patent Application Laid-Open No. 2-109985, it is possible to incorporate the phosphofructokinase-encoding gene into transposon, and allow it to be transferred to introduce the multiple copies into the chromosomal DNA. By either method, the number of copies of the phosphofructokinase-encoding gene in the transformant strain increases, and as a result, the phosphofructokinase activity is increased.

The increase of the phosphofructokinase activity can also be obtained by, besides being based on the aforementioned gene amplification, replacing an expression regulatory sequence such as a promoter of the phosphofructokinase-encoding gene on the chromosome DNA or plasmid with a strong one. For example, *lac* promoter, *trp* promoter, *trc* promoter, *tac* promoter, P_{R} promoter and P_{L} promoter of lambda phage. Replacement with these promoters increases expression of the phosphofructokinase-encoding gene, and as a result, the phosphofructokinase activity is increased.

In the coryneform bacteria of the present invention, in addition to the phosphofructokinase activity, activity of another enzyme of the L-lysine biosynthesis pathway, the glycolytic pathway or the like may be increased by introduction or amplification of the gene thereof. As examples of the gene usable in L-lysine production, a gene encoding α-subunit or β-subunit of aspartokinase substantially desensitized in synergistic feedback inhibition by L-lysine and L-threonine (International Publication No. WO 94/25605), a wild type phosphoenolpyruvate carboxylase gene originating from a coryneform bacterium (Japanese Patent Application Laid-Open No. 60-87788), a gene encoding wild type dihydrodipicolinate synthase originating from a coryneform bacterium (Japanese Patent Publication No. 6-55149) and the like are known.

Further, activity of an enzyme catalyzing reaction of producing a compound other than L-lysine and branching from the L-lysine biosynthesis pathway, may be decreased or deficient. As the enzyme catalyzing reaction of producing a compound other than L-lysine and branching from the L-lysine biosynthesis pathway, homoserine dehydrogenase may be mentioned (see WO 95/23864).

In this specification, the expression of "(enzyme) activity is increased" means that the intracellular enzymatic activity is higher that that of a wild strain, and that the intracellular enzymatic activity is higher than that of a strain before modification when a strain in which the intracellular activity is increased by modification by genetic recombination technology or the like is obtained. Also, the expression of "(enzyme) activity is decreased" means that the intracellular enzymatic activity is lower that that of a wild strain, and that the intracellular enzymatic activity is lower than that of a strain before modification when a strain in which the intracellular activity is decreased by modification by genetic recombination technology or the like is obtained.

### <3> Production of L-lysine

By culturing the coryneform bacterium in which an intracellular activity of phosphofructokinase is increased, and which has L-lysine productivity, in an appropriate medium, L-lysine is accumulated in the medium.

The medium to be used for L-lysine production by using the microorganism of the present invention may be an ordinary medium containing a carbon source, a nitrogen source, inorganic ions, and optionally other organic micronutrients. As the carbon source, carbohydrates such as glucose, lactose, galactose, fructose, sucrose, molasses, and starch hydrolysate; alcohols such as ethanol and inositol, and organic acids such as acetic acid, fumaric acid, citric acid, and succinic acid may be used.

As the nitrogen source, inorganic ammonium salts such as ammonium sulfate, ammonium nitrate, ammonium chloride, ammonium phosphate and ammonium acetate; organic nitrogen such as ammonia, peptone, meat extract, yeast extract, yeast extract, yeast extract, corn steep liquor, and soybean hydrolysate; ammonia gas; and aqueous ammonia may be used.

As the inorganic ions, potassium phosphate, magnesium sulfate, iron ion, manganese ion and so on may be added in small amounts. As organic micronutrients, it is desirable to contain required substances such as vitamin B₁ or yeast extract or the like in appropriate amounts.

Cultivation is preferably carried out under an aerobic condition by shake culture, aeration agitation culture or the like for about 16 to 72 hours. The cultivation temperature is usually controlled at 30°C to 45°C, and pH is usually controlled at 5 to 9 during cultivation. Inorganic or organic, acidic or alkaline substances, or ammonia gas or the like can be used for pH adjustment.

L-Lysine can be collected from a culture by combining an ordinary ion exchange resin method, a precipitation method, and other known methods.

### EXAMPLES

The present invention will be more specifically explained below with reference to Examples.

### Example 1

### Introduction of Escherichia coli pfkB gene into L-lysine-producing strain of coryneform bacterium, and production of L-lysine

### <1> Cloning of pfkB gene of Escherichia coli JM109

The nucleotide sequence of the *pfkB* gene of *Escherichia coli* has already been elucidated (Daldal, F., Gene, 28, 337-342 (1984); Daldal, F., J. Mol. Biol., 168,, 285-305 (1983); GenBank/EMBL/DDBJ accession No. K00128). Primers shown in SEQ ID Nos: 1 and 2 in the Sequence Listing were synthesized based on the reported nucleotide sequence, and PCR was performed by using chromosomal DNA of *Escherichia coli* as a template to amplify a phosphofructokinase gene.

Among the synthesized primers, SEQ ID NO: 1 corresponds to a sequence of from 1^{st} base to 24^{th} base, and SEQ ID NO: 2 corresponds to a sequence of from 1268^{th} base to 1245^{th} base in the sequence of the *pfkB* gene described in Daldal, F., Gene, 28, 337-342 (1984).

The preparation of the chromosomal DNA of *Escherichia coli* was performed according to an ordinary method (Seibutsu Kogaku Jikkensho, The Society for Bioscience and Bioengineering, Japan ed., 97-98, Baifukan, 1992). Also, for the PCR reaction, the standard reaction condition described on page 185 of PCR Hou Saizensen (Takeo Sekiya et al. ed., Kyoritsu Shuppan, 1989) was used.

After the produced PCR product was purified according to an ordinary method, it was ligated with plasmid pHC4 (see Japanese Patent Application Laid-Open No. 5-7491) by using a ligation kit (Takara Shuzo) and then competent cells of *Escherichia coli* JM109 (Takara Shuzo) were transformed therewith, spread on L-medium (10 g/L Bacto trypton, 5 g/L Bacto yeast extract, 5 g/L NaCl, 15 g/L agar, pH 7.2) containing 5 µg/ml chloramphenicol, and cultured overnight. Appeared white colonies were picked up and subjected to single colony isolation to obtain a transformant. Plasmid was extracted from the obtained transformant to obtain plasmid pHC4pfk in which the *pfkB* gene is ligated to the vector.

The *Escherichia coli* harboring pHC4 was designated as private number AJ12617, and deposited at National Institute of Bioscience and Human-Technology, Agency of Industrial Science and Technology, Ministry of International Trade and Industry (postal code 305-8566, 1-3 Higashi 1-chome, Tsukuba-shi, Ibaraki-ken, Japan) on April 24, 1991 and received an accession number of FERM P-12215, and it was transferred to an international deposition under the provisions of the Budapest Treaty on August 26, 1991, and received an accession number of FERM BP-3532.

In order to confirm that the cloned DNA fragment encodes a protein having phosphofructokinase activity, phosphofructokinase activities of the strain JM109 and the strain harboring pHC4pfk were determined by the method described in Bloxham, D.P. and Lardy, H.A., The Enzymes (3^{rd} ed.), 8, 239-278 (1973). As a result, the strain JM109 harboring pHC4pfk showed phosphofructokinase activity of 15 times higher than that of the strain JM109 not harboring pHC4pfk, whereby expression of the *pfkB* gene was confirmed.

### <2> Introduction of pHC4pfk into L-lysine-producing strain of coryneform bacterium, and L-lysine production

*Brevibacterium lactofermentum* AJ11082 was transformed with plasmid pHC4pfk according to the electric pulse method (Japanese Patent Application Laid-Open No. 2-207791), to obtain a transformant.

By using the obtained transformant AJ11082/pHC4pfk, cultivation for L-lysine production was performed as follows. Cells of the strain AJ11082/pHC4pfk obtained by culturing on CM2B plate medium containing 5 µg/ml chloramphenicol were inoculated to the L-lysine-producing medium having the composition described below and containing 5 µg/ml chloramphenicol, to perform shake culture at 31.5°C until sugar in the medium was consumed. As a control, the *Corynebacterium* strain AJ11082 was transformed with already obtained plasmid pHC4 autonomously replicable in *Corynebacterium* bacteria according to the electric pulse method, and cultured similarly to the above.

The *Brevibacterium lactofermentum* AJ11082 was deposited at Agricultural Research Culture Collection (1815 N. University Street, Peoria, Illinois 61604 U.S.A.) on January 31, 1981 and received an accession number of NRRL B-11470.

### [L-Lysine-producing medium]

The following components other than calcium carbonate (per liter) were dissolved and pH was adjusted to 8.0 with KOH. The medium was sterilized at 115°C for 15 minutes, and calcium carbonate (50 g) separately subjected to dry heat sterilization was added to the sterilized medium.

| | |
|---|---|
| Glucose | 100 g |
| (NH₄)₂SO₄ | 55 g |
| KH₂PO₄ | 1 g |
| MgSO₄ · 7H₂O | 1 g |
| Biotin | 500 µg |
| Thiamin | 2000 µg |
| FeSO₄ · 7H₂O | 0.01 g |
| MnSO₄ · 7H₂O | 0.01 g |
| Nicotinamide | 5 mg |
| Protein hydrolysate (Mamenou) | 30 ml |
| Calcium carbonate | 50 g |

After completion of the cultivation, an accumulated amount of L-lysine in the culture medium was determined with Biotec Analyzer AS-210 (Asahi Kasei Kogyo). The results are shown in Table 1.

**Table 1**

| Strain | Accumulated amount of L-lysine (g/L) |
|---|---|
| AJ11082/pHC4 | 29.9 |
| AJ11082/pHC4pfk | 32.8 |

### Example 2

### Cloning of pfk gene from Brevibacterium lactofermentum

### <1> Obtainment of partial fragment of pfk gene of Brevibacterium lactofermentum ATCC 13869

A region having a high homology in amino acid sequences between phosphofructokinases encoded by a *pfk* gene from *Streptomyces coelicolor* (Appl. Environ. Microbiol. 63(3), 956-961 (1997)) and a *pfkA* gene from *Escherichia coli* (Eur. J. Biochem. 149(2), 363-373 (1985)) as known *pfk* genes was selected. From the region, nucleotide sequences were deduced to synthesize oligonucleotides shown in SEQ ID NO: 3 and SEQ ID NO: 4.

Chromosome DNA of *Brevibacterium lactofermentum* ATCC 13869 was prepared by using Bacterial Genome DNA Purification Kit (Advanced Genetic Technologies Corp.). A PCR mixture in a total volume of 50 µl was prepared by adding sterilized water to 0.5 µg of the chromosome DNA, 20 pmol each of the oligonucleotides, 4 µl of dNTP mixture (2.5 mM each), 5 µl of 10X ExTaq Buffer (Takara Shuzo) and 1 U of ExTaq (Takara Shuzo).

With respect to the PCR mixture, PCR was performed with Thermal Cycler TP240 (Takara Shuzo) under condition of 30 cycles of denaturation, 94°C, 30 seconds; annealing, 37 to 60°C, 15 seconds; and extension, 72°C, 45 seconds. Agarose gel electrophoresis of the amplified products showed that the reaction mixture contained a band of about 460 bp when annealing temperature was 45°C.

The reaction product contained in the reaction mixture when annealing temperature was 45°C, was ligated to pCR2.1 (in vitro gen.) by using Original TA Cloning Kit (in vitro gen.). Competent cells of *Escherichia coli* JM109 (Takara Shuzo) were transformed with the ligation product, spread on L-medium (10 g/L Bacto trypton, 5 g/L Bacto yeast extract, 5 g/L NaCl, 15 g/L agar, pH 7.2) containing 10 µg/ml IPTG (isopropyl-β-D-thiogalactopyranoside), 40 µg/ml X-Gal (5-bromo-4-chloro-3-indolyl-β-D-thiogalactopyranoside), 25 µg/ml kanamycin, and cultured overnight. Appeared white colonies were picked up and subjected to single colony isolation to obtain a transformant.

From the transformant, plasmid was prepared according to the alkali method (Seibutsu Kogaku Jikkensho, The Society for Bioscience and Bioengineering, Japan ed., p. 105, Baifukan, 1992). Nucleotide sequences at both ends of the inserted fragment was determined according to Sanger's method (J. Mol. Biol., 143, 161 (1980)) by using oligonucleotides shown in SEQ ID NO: 5 and SEQ ID NO: 6. Specifically, Bigdye terminator sequencing kit (Applied Biosystems) was used for sequencing, and the sequence was analyzed with Genetic Analyzer ABI310 (Applied Biosystems). The determined nucleotide sequence was translated to an amino acid sequence. Comparison of the amino acid sequence with those deduced from the *pfk* genes from *Streptomyces coelicolor* and *Escherichia coli* showed a high homology. Therefore, it was decided that the cloned fragment in pCR2.1 was a *pfk* gene from *Brevibacterium lactofermentum*.

### <2> Determination of entire nucleotide sequence of pfk gene from Brevibacterium lactofermentum

The fragment contained in the plasmid prepared in the above <1> was a partial fragment of the *pfk* gene, and it was necessary to determine the nucleotide sequence of the full-length *pfk* gene. As a method for determining an unknown nucleotide sequence flanking a known region, the inverse PCR method (Genetics 120, 621-623 (1988)), a method using the LA-PCR in vitro cloning kit (Takara Shuzo) or the like is known. In this Example, determination of the unknown nucleotide sequence was determined according to the inverse PCR method as follows.

Based on the nucleotide sequence determined in the above <1>, oligonucleotides shown in SEQ ID NO: 7, SEQ ID NO: 8, SEQ ID NO: 9 and SEQ ID NO: 10 were synthesized. Separately, the chromosome DNA of *Brevibacterium lactofermentum* was completely digested with each of *Eco*RI, *Bam*HI, *Kpn*I, *Hind*III, *Pst*I, *Sph*I, *Sac*I and *Sal*I, and the digested products were cyclized by intramolecular ligation reaction by using Ligation kit Ver. 2 (Takara Shuzo). A PCR mixture in a total volume of 50 µl was prepared by adding sterilized water to 0.5 µg of the cyclized chromosome DNA, 10 pmol each of the oligonucleotides of SEQ ID NO: 7 and SEQ ID NO: 8, 4 µl of dNTP mixture (2.5 mM each), 5 µl of 10X ExTaq Buffer (Takara Shuzo) and 1 U of ExTaq (Takara Shuzo). With respect to the PCR mixture, PCR was performed with Thermal Cycler TP240 (Takara Shuzo) under condition of 30 cycles of denaturation, 94°C, 30 seconds; annealing, 55°C, 15 seconds; and extension, 72°C, 3 minutes.

The PCR products were analyzed by agarose gel electrophoresis, but no amplified product was clearly observed if any of restriction enzymes was used. Therefore, nested PCR was further performed (Technique, 1, 165-170 (1989)). A PCR mixture in a total volume of 50 µl was prepared by adding sterilized water to 1 µl of each of the PCR reaction mixtures diluted by 1,000 times with sterilized water, 10 pmol each of the oligonucleotides of SEQ ID NO: 9 and SEQ ID NO: 10, 4 µl of dNTP mixture (2.5 mM each), 5 µl of 10X ExTaq Buffer (Takara Shuzo) and 1 U of ExTaq (Takara Shuzo). With respect to the PCR mixture, PCR was performed with Thermal Cycler TP240 (Takara Shuzo) under condition of 30 cycles of denaturation, 94°C, 30 seconds; annealing, 55°C, 15 seconds; and extension, 72°C, 3 minutes.

The PCR reaction mixtures were subjected to agarose gel electrophoresis. As a result, a band of about 2000 bp was observed when the product obtained by digestion with *Pst*I and intramolecular ligation reaction was used as a template. This band was purified by using Suprec ver. 2 (Takara Shuzo), and the nucleotide sequence of the *pfk* gene contained in the PCR product of 2000 bp was determined in the similar way to <1> by using oligonucleotides shown in SEQ ID NO: 9 and SEQ ID NO: 10.

The nucleotide sequence of about 1300 bp containing the *pfk* gene in the thus determined nucleotide sequence is shown in SEQ ID NO: 13 in Sequence Listing. An amino acid sequence obtained by translation of an open reading frame predicted from the nucleotide sequence is shown in SEQ ID NO: 12. The protein having the amino acid sequence shown in SEQ ID NO: 12 is phosphofructokinase from *Brevibacterium lactofermentum*. Because the methionine residue existing on the N-terminus end of a protein originating from the initiation codon, ATG, the residue is often unrelated to the essential function of the protein. Also, it is known that the residue is often removed by action of peptidase after translation. In the above protein, the removal of the methionine residue may occur.

With respect to each of the nucleotide sequence and the amino acid sequence, homology comparison to known sequences was performed. Used databases were GenBank and SWISS-PROT. As a result, it have been revealed that the DNA shown in SEQ ID NO: 11 is a gene encoding a protein which has homology to PFK encoded by already reported *pfkA* of *Escherichia coli*, and is novel in coryneform bacteria.

### Industrial Applicability

According to the present invention, the L-lysine productivity of a coryneform bacterium can be improved, thereby providing a more efficient method for producing L-lysine. Also, the gene encoding phosphofructokinase of *Brevibacterium lactofermentum* of the present invention can be suitably used for breeding L-lysine-producing coryneform bacteria.

## Claims

1. A coryneform bacterium in which an intracellular activity of 6-phosphofructokinase is increased, and which has L-lysine productivity.

2. The coryneform bacterium according to claim 1, wherein the intracellular activity of 6-phosphofructokinase is increased by increasing a number of copies of a gene encoding 6-phosphofructokinase in a cell of the bacterium.

3. The coryneform bacterium according to claim 2, wherein the gene encoding 6-phosphofructokinase originates from a coryneform bacterium.

4. A method for producing L-lysine comprising the steps of cultivating said coryneform bacterium as defined in any one of claims 1 to 3 in a medium, to produce and accumulate L-lysine in a culture, and collecting L-lysine from the culture.

5. A DNA which encodes a protein defined in the following (A) or (B):
(A) a protein which has the amino acid sequence of SEQ ID NO: 12, or
(B) a protein which has an amino acid sequences of SEQ ID NO: 12 including substitution, deletion, insertion, addition or inversion of one or several amino acids, and has 6-phosphofructokinase activity.

6. The DNA according to claim 5, which is a DNA defined in the following (a) or (b):
(a) a DNA which has a nucleotide sequence comprising the nucleotide sequence of the nucleotide numbers 116 to 1153 of SEQ ID NO: 11; or
(b) a DNA which is hybridizable with a probe having the nucleotide sequence of the nucleotide numbers 116 to 1153 of SEQ ID NO: 11 or a part thereof under a stringent condition, and encodes a protein having 6-phosphofructokinase activity.
